# EUROPEAN PATENT APPLICATION

(11) **EP 4 578 374 A1**
(43) Date of publication of application: **02.07.2025**
(21) Application number: 23857283.8
(22) Date of filing: 17.08.2023
(51) Int. Cl.: A61B 1/06, G02B 23/24

(54) **LIGHT SOURCE DEVICE AND ENDOSCOPE SYSTEM**

(30) Priority: 23.08.2022 JP 2022132213
(71) Applicant: HOYA Corporation, Tokyo 160-8347 (JP)
(72) Inventor: HAYASHI Yoshihiro, Tokyo 160-8347 (JP)
(74) Representative: Schaumburg und Partner Patentanwälte mbB
(86) International application number: PCT/JP2023/029733
(87) International publication number: WO 2024/043171

(57) **Abstract**

The present disclosure proposes a light source device for generating illumination light to be applied to an object, the light source device ensuring a sufficient light amount while avoiding the occurrence of artifacts and distortion due to a rolling shutter, and making brightness unevenness and horizontal stripes less noticeable even if a change in a pulsed light emission organ extends to a rolling shutter period. The light source device includes a semiconductor light emitting element that emits light, and a light source control unit that generates an emission profile of the semiconductor light emitting element and drives the semiconductor light emitting element on the basis of the emission profile. The light source control unit executes: processing of dividing one frame period in an image signal acquired by an image sensor into N (N is an integer of 2^{m}, m being an integer of 2 or more) dimming sections; processing of generating an emission profile for controlling light emission intensity in units of 2^{k} (k is an integer from 0 to m) dimming sections according to a target light amount in one frame period; and processing of performing dimming control for a next frame on the basis of the emission profile (see Fig. 7B).

## Description

### Technical Field

The present disclosure relates to a light source device and an endoscope system.

### Background Art

A normal endoscope device equipped with a rolling shutter type image sensor executes pseudo-global exposure to avoid the occurrence of undesirable phenomena caused by a rolling shutter, such as distortion and artifacts, by turning off a light source in a valid pixel reading period (rolling shutter period) of the image sensor, and turning on the light source in other period (pseudo-global exposure period) (pulsed light emission control).

On the other hand, when the light source is completely turned off during the rolling shutter period, a light amount becomes insufficient depending on an object (site to be observed), and a satisfactory image cannot be acquired. In order to solve the insufficiency of the light amount, Patent Literatures 1 to 3 and the like, for example, describe light source control in which a part of a rolling shutter period is included in a pulsed light emission period.

### Citation List

### Patent Literature

Patent Literature 1: JP 2018-182580 A
Patent Literature 2: JP 5379932 B1
Patent Literature 3: JP 6239220 B1

### Summary of Invention

### Technical Problem

However, when the light source control as described in Patent Literatures 1 to 3 is executed, brightness unevenness, horizontal stripes, and the like, of a screen occur due to a difference in exposure period for each line in adjacent frames. There is a problem that, due to a change in the pulsed light emission period for each frame, the brightness unevenness and the horizontal stripes move up and down on a display screen, which is annoying. In addition, in a case where offset light emission is performed during the rolling shutter period in order to solve the insufficiency of the light amount, the offset light emission that increases to a certain level results in an unnatural image like an image generated by double exposure of a long-time exposure image and a high-speed exposure image.

The present disclosure has been made in view of such a situation and proposes a technique of ensuring a sufficient light amount while avoiding the occurrence of distortion and artifacts caused by a rolling shutter and making brightness unevenness and horizontal stripes less noticeable even if a change in a pulsed light emission period extends to a rolling shutter period.

### Solution to Problem

In order to address the above-mentioned problem, the present embodiment propose a light source device that generates illumination light to be applied to an object, the light source device including: a semiconductor light emitting element that emits light; and a light source control unit that generates an emission profile of the semiconductor light emitting element and drives the semiconductor light emitting element on the basis of the emission profile, wherein the light source control unit executes processing of dividing one frame period in an image signal acquired by an image sensor into N (N is an integer of 2^{m}, m being an integer of 2 or more) dimming sections, processing of generating an emission profile for controlling light emission intensity in units of 2^{k} (k is an integer from 0 to m) dimming sections included in the dimming sections according to a target light amount in the one frame period, and processing of performing dimming control for a next frame on the basis of the emission profile.

The present embodiment also proposes an endoscope system that inserts an endoscope device into a site to be observed and acquires an image of an object, the endoscope system including: a light source device; an image sensor that irradiates the object with illumination light emitted from the light source device and generates an image signal by detecting reflection light from the object; and a processor that processes the image signal to generate an image of the object and displays the generated image on a monitor, wherein the light source device includes a semiconductor light emitting element that emits light, and a light source control unit that generates an emission profile of the semiconductor light emitting element and drives the semiconductor light emitting element on the basis of the emission profile, and the light source control unit executes processing of dividing one frame period in an image signal acquired by the image sensor into N (N is an integer of 2^{m}, m being an integer of 2 or more) dimming sections, processing of generating an emission profile for controlling light emission intensity in units of 2^{k} (k is an integer from 0 to m) dimming sections included in the dimming sections according to a target light amount in the one frame period, and processing of performing dimming control for a next frame on the basis of the emission profile.

Further features related to the present disclosure will become apparent from the description of the present specification and the accompanying drawings. The present disclosure is achieved and implemented by elements and combinations of various elements and by modes of the following detailed description and the appended claims.

The description in this specification is merely exemplary and is not intended to limit the scope of the claims or application examples of the present disclosure in any sense.

### Advantageous Effects of Invention

According to the present disclosure, it is possible to ensure a sufficient light amount while avoiding the occurrence of distortion and artifacts caused by a rolling shutter, and to make brightness unevenness and horizontal stripes less noticeable even when a change in a pulsed light emission organ extends to a rolling shutter period.

### Brief Description of Drawings

Fig. 1 is a diagram illustrating an overall external appearance example of an endoscope system according to the present embodiment.
Fig. 2 is a diagram schematically illustrating an internal configuration example of the endoscope system according to the present embodiment.
Fig. 3 is a diagram illustrating an internal configuration example of a light source device 101 provided inside a processor 200.
Fig. 4 is a diagram illustrating spectra (wavelength characteristics) of LEDs 1011 to 1015.
Fig. 5 is a diagram illustrating characteristics of illumination light (light illuminating an observation site) generated by transmitting light from each LED through cross prisms 1017 and 1018.
Fig. 6 is a diagram illustrating a valid pixel region and an invalid region of a rolling shutter type image sensor that is a CMOS sensor as an example.
Fig. 7A is a diagram (part 1) for describing an outline of dimming control processing 1 (Example 1) according to the present embodiment.
Fig. 7B is a diagram (part 2) for describing the outline of dimming control processing 1 (Example 1) according to the present embodiment.
Fig. 8 is a flowchart for describing details of processing for generating an emission profile based on the dimming control processing 1 (Example 1) according to the present embodiment.
Fig. 9A is a diagram (part 1) for describing an outline of dimming control processing 2 (Example 2) according to the present embodiment.
Fig. 9B is a diagram (part 2) for describing the outline of dimming control processing 2 (Example 2) according to the present embodiment.
Fig. 10 is a flowchart for describing details of processing for generating an emission profile based on the dimming control processing 1 (Example 2) according to the present embodiment.

### Description of Embodiments

Embodiments of the present disclosure will be described below with reference to the drawings. In the following, an endoscope system will be described as an embodiment of the present disclosure.

Examples of a site to be observed by the endoscope system include respiratory organs and digestive organs. Examples of the respiratory organs include the lungs, the bronchus, the ears, the nose, and the throat. Examples of the digestive organs include the large intestine, the small intestine, the stomach, the esophagus, the duodenum, the uterus, and the bladder. When the above-mentioned sites are observed, it is more effective to utilize an image in which a specific biological structure is emphasized.

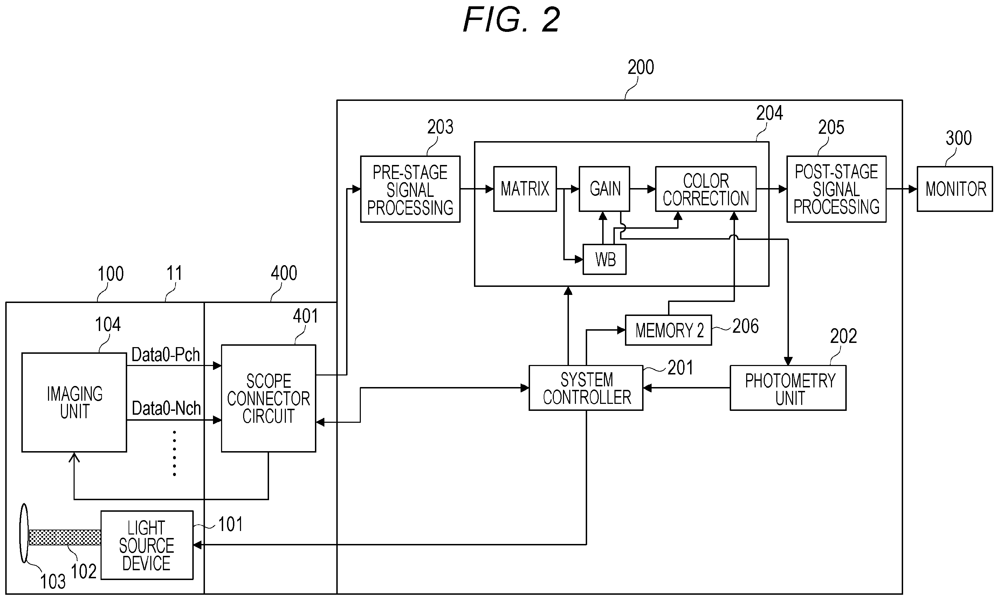

Note that the technology disclosed in the present embodiment can also be applied to a configuration in which a light source device is provided in a processor, but can also be applied to a configuration in which a light source device is provided in an internal space of an endoscope device that is a volumetrically limited and closed space. Therefore, in the present embodiment, the latter configuration will be mainly described (see Fig. 2).

### <Positioning of present technology>

The present inventor has filed Japanese Patent Application No. 2020-179317 (filed on October 27, 2020) (JP 2022-070310 A) as a technology related to the present technology. The prior application discloses, in order to avoid scanning line noise generated at the time of switching from pulsed light to continuous light and a double exposure-like image generated at the time of performing light emission intensity control in a rolling shutter period, performing intensity-time reduction processing of controlling the light emission intensity during the rolling shutter period to address a change in brightness of an image and gradually reducing the light emission intensity to a light emission time. The intensity-time reduction processing is a process of converting the light emission time into the light emission intensity on a frame basis with the total area of an emission profile representing a light amount integrated value being kept constant. In order to perform the processing, a complicated operation such as logarithmic operation or floating point multiplication needs to be executed on a frame basis. When sufficient resources are provided such as a processor, there is no particular inconvenience. However, calculation resources are not sufficient as in a case where, for example, a light source device is provided on the endoscope device side. For this reason, it is conceivable to add a resource (for example, a CPU or the like) for the above-described complicated calculation. However, since the endoscope device has a closed space for ensuring waterproofness, there is also a risk that the operation performed by an operator is affected by generated heat when the above-described complicated calculation is executed by the CPU. In addition, the device may have a temperature exceeding the rated temperature of the device, which may lead to a dangerous situation. In order to avoid such a situation, it is conceivable to provide a special heat dissipation mechanism. However, providing the heat dissipation mechanism leads to an increase in manufacturing cost of the device, which is not necessarily preferable. Therefore, it is desirable that a light source control unit (FPGA) of the light source device completes the dimming control. However, unlike the CPU, the light source control unit is not suitable for executing complicated calculation, and thus, it is necessary to simplify calculation for dimming control.

In view of such a situation, the present embodiment will describe dimming control processing that has an operation amount much smaller than that of the above-described intensity-time reduction processing, can be executed by a light source control unit (FPGA), and can avoid the occurrence of scanning line noise and a double exposure-like image as in the above-described intensity-time reduction processing.

### <Configuration of endoscope system>

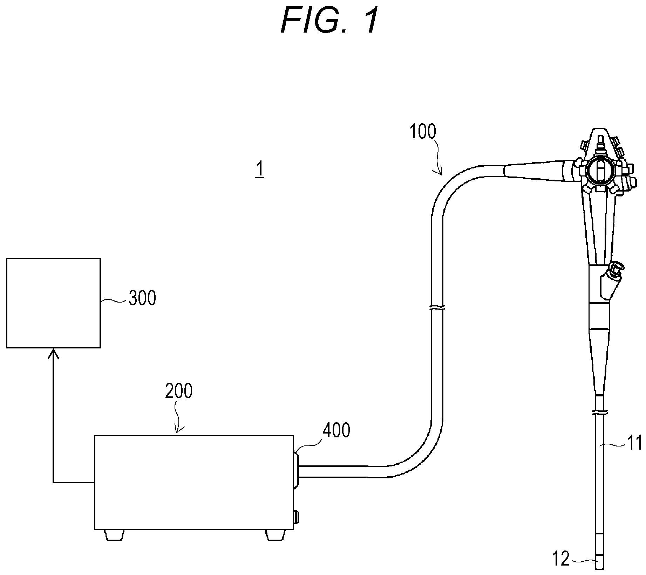

Fig. 1 is a diagram illustrating an example of an overall external appearance of an endoscope system according to the present embodiment, and Fig. 2 is a diagram schematically illustrating an internal configuration example of the endoscope system according to the present embodiment. An endoscope system 1 includes an endoscope device (electronic scope) 100, a processor 200, and a monitor 300. Note that a scope connector (which may hereinafter be simply referred to as a "connector") 400 including a connector circuit according to the feature of the present embodiment is provided at a processor-side end portion of the endoscope device 100.

The endoscope device 100 includes an elongated tubular insertion portion 11 to be inserted into a subject. The endoscope device 100 includes, for example, a light source device 101, a light carrying bundle (LCB) 102 for guiding irradiation light from the light source device 101, a light distribution lens 103 provided at an emission end of the LCB 102, an imaging unit 104 that receives return light from an irradiated portion (observation site) via an objective lens (not illustrated), a driver signal processing circuit (not illustrated) that drives the imaging unit 104, and a first memory (not illustrated).

The irradiation light from the light source device 101 enters the LCB 102 and propagates by repeating total reflection in the LCB 102. The irradiation light (illumination light) propagating in the LCB 102 is emitted from the emission end of the LCB 102 disposed in a distal end portion 12 of the insertion portion 11 and irradiates the observation site through the light distribution lens 103. The return light from the irradiated portion forms an optical image at each pixel on a light receiving surface of the imaging unit 104 via the objective lens.

The imaging unit 104 is disposed in the distal end portion 12 of the insertion portion 11 and can use a complementary metal oxide semiconductor (CMOS) image sensor which is a rolling shutter type image sensor. The imaging unit 104 accumulates optical images (return light from a biological tissue) formed at each pixel on the light receiving surface as electric charges corresponding to a light amount, and generates and outputs image signals of R, G, and B. Note that the imaging unit 104 is not limited to the CMOS image sensor and may be replaced with another type of imaging device as long as it is based on the rolling shutter type. A signal output from the imaging unit 104 is processed by a scope connector circuit 401 provided in the scope connector 400 as will be described later.

The processor 200 is a device that integrally includes a signal processing device that processes a signal from the endoscope device 100 and a light source device that irradiates, via the endoscope device 100, a body cavity where natural light cannot reach. In another embodiment, the signal processing device and the light source device may be provided separately. The processor 200 includes a system controller 201, a photometry unit 202, a pre-stage signal processing circuit 203, a color conversion circuit 204, a post-stage signal processing circuit 205, and a second memory 206.

The processor 200 may include an operation panel (not illustrated). There are various forms in a configuration of the operation panel. Examples of a specific configuration of the operation panel include a hardware key for each function mounted on a front surface of the processor 200, a touch panel type graphical user interface (GUI), and a combination of the hardware key and the GUI. An operator (surgeon) can perform a mode switching operation that will be described later with the operation panel.

The photometry unit 202 acquires luminance information of an image signal obtained through imaging from a gain circuit included in the color conversion circuit 204, compares the acquired luminance information with a predetermined appropriate luminance value (for example, information regarding the appropriate luminance value may be stored in advance in an internal memory (not illustrated) of the photometry unit 202), and notifies the system controller 201 of a comparison result (whether a current luminance value is appropriate, higher, or lower).

The system controller 201 executes various programs stored in a memory (not illustrated) and controls the entire endoscope system 1 in an integrated manner. The system controller 201 controls operations and timings of various circuits in the processor 200 by using a control signal to perform processing suitable for the endoscope device 100 connected to the processor 200. The system controller 201 may be connected to the above-described operation panel.

The system controller 201 receives the comparison result with the appropriate luminance value from the photometry unit 202, determines whether to maintain current exposure (exposure), whether to increase the exposure (including a level value to increase), or whether to decrease the exposure (including a level value to decrease), and outputs the comparison result to the light source device 101 as an exposure control signal.

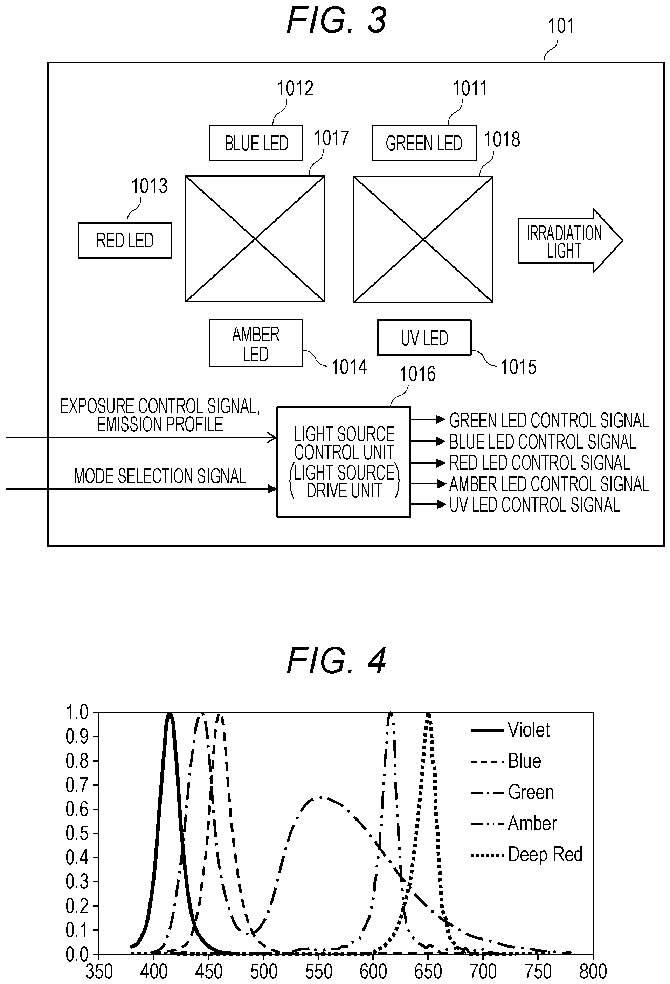

The system controller 201 changes each operation of the endoscope system 1 and parameters for the corresponding operation in accordance with an operator's instruction input from the operation panel. For example, when the operator selects an observation mode with the operation panel (mode switching operation), the system controller 201 outputs a mode selection signal for causing a light source corresponding to the observation mode to emit light to the light source device 101. As will be described later, a plurality of light emitting diodes (LEDs) that emit light with different wavelength bands, for example, may be used as the light source device 101 (see Fig. 3). When the operator selects the observation mode (for example, a normal observation mode, a special light observation mode, a SatO2 mode, or the like) by operating a mode selection switch provided in the processor 200, for example, the system controller 201 generates a mode selection signal corresponding to the selected mode and supplies the generated mode selection signal to a light source control unit (light source control circuit (FPGA)) 1016 of the light source device 101 (see Fig. 3). On the basis of the mode selection signal, the light source control unit 1016 determines a combination of LEDs that will emit light and their intensity and light amounts (for example, a combination, and the like, of light emitting LEDs corresponding to the mode selection signal are stored in advance in an internal memory (not illustrated)), and outputs a necessary LED control signal from the LEDs 1011 to 1015. When the LEDs 1011 to 1015 emit light beams of wavelength bands based on the LED control signal supplied from the light source control unit 1016, the emitted light beams are synthesized by a cross prism to generate irradiation light (synthesized light).

The endoscope device 100 and the processor 200 may perform data communication using a wired electric communication method or an optical wireless communication method.

As illustrated in Fig. 2, the endoscope device 100 and the processor 200 are connected via the scope connector 400. The connector 400 includes a scope connector circuit 401. The scope connector circuit 401 is provided in the scope connector 400 in the present embodiment, but is not necessarily provided in the scope connector 400. For example, a circuit corresponding to the scope connector circuit 401 may be provided in a connector unit on the processor 200 side or inside the processor 200.

### <Internal configuration example of light source device 101>

Fig. 3 is a diagram illustrating an internal configuration example of the light source device 101 provided inside the endoscope device 100, for example.

The light source device 101 includes a green LED 1011 that emits green light, a blue LED 1012 that emits blue light, a red LED 1013 that emits red light, an amber LED 1014 that emits amber light, a UV LED 1015 that emits UV light, a light source control unit 1016 that controls light emission of the LEDs 1011 to 1015, and cross prisms 1017 and 1018.

When receiving the exposure control signal from the system controller 201, the light source control unit 1016 which can be configured from, for example, FPGA changes an emission profile of each LED to adjust exposure (adjust light amount) by controlling a light emission period and an applied current value of each LED that is currently emitting light (a combination of LEDs that will emit light is determined depending on the observation mode). For example, after changing the emission profile by one step, the light source control unit 1016 determines whether to change the above-described emission profile again to adjust exposure (perform dimming control) on the basis of an exposure control signal determined from a photometry result (a comparison result with the appropriate luminance value) from the photometry unit 202.

The light source control unit 1016 determines a combination of LEDs that are to emit light on the basis of a mode selection signal indicating an observation mode selected by the operator. In a light emission start stage, the light source control unit 1016 controls light emission of each LED on the basis of, for example, a predetermined emission profile (a default light emission period and drive current value), and thereafter, performs exposure adjustment as described above.

### <Each LED light source>

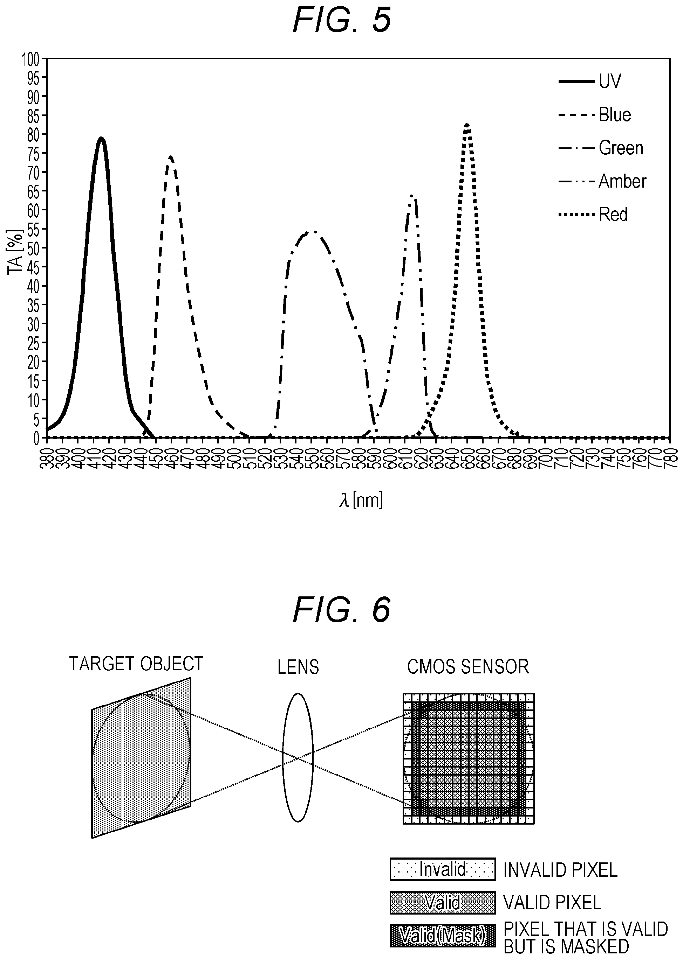

Fig. 4 is a diagram illustrating spectra (wavelength characteristics) of the LEDs 1011 to 1015. Fig. 5 is a diagram illustrating characteristics of illumination light (light illuminating an observation site) generated by transmitting light from each LED through the cross prisms 1017 and 1018.

The green LED 1011 has a transmission wavelength band of 540 nm to 575 nm, a peak wavelength of 550 nm, and a half width of 30 nm. The green LED 1011 is provided with a phosphor and emits light in a transmission wavelength band of about 400 nm to 780 nm due to the phosphor as illustrated in Fig. 4. In other words, white light is substantially emitted by the green LED and the phosphor, and this white light is an intermediate product. As will be described later, the transmission wavelength band of the white light is narrowed by the cross prism 1018, and thus, an observation site is irradiated with green light. The blue LED 1012 has a transmission wavelength band of 460 nm to 490 nm, a peak wavelength of 456 nm, and a half width of 21 nm. The red LED 1013 has a transmission wavelength band of 630 nm to 1000 nm, a peak wavelength of 650 nm, and a half width of 20 nm. The amber LED 1014 has a transmission wavelength band of 600 nm to 615 nm, a peak wavelength of 613 nm, and a half width of 19 nm. The UV LED 1015 has a transmission wavelength band of 385 nm to 425 nm, a peak wavelength of 405 nm, and a half width of 14 nm.

The light beams (white light beam, blue light beam, red light beam, amber light beam, and UV light beam as intermediate products) generated from the LEDs 1011 to 1015 including the green LED 1011 on which the phosphor is mounted are converted into light beams having characteristics illustrated in Fig. 5 after transmitting through the cross prisms 1017 and 1018, and are applied to the observation site. Specifically, the white light generated from the green LED 1011 and the phosphor is limited in terms of a transmission wavelength band by the cross prism 1018, and is converted into green light with 520 nm to 595 nm. The blue light emitted from the blue LED 1012 is converted into blue light with 440 nm to 500 nm by the cross prisms 1017 and 1018. The red light emitted from the red LED 2013 is converted into red light with 620 nm to 630 nm by the cross prisms 1017 and 1018. The amber light emitted from the amber LED 1014 is converted into amber light with 580 nm to 630 nm by the cross prisms 1017 and 1018. The UV light emitted from the UV LED 1015 is converted into UV light with 380 nm to 450 nm by the cross prism 1018.

### <Configuration example of imaging surface of image sensor>

Fig. 6 is a diagram illustrating a valid pixel region and an invalid region of a rolling shutter type image sensor that is a CMOS sensor as an example. The CMOS sensor includes a valid pixel region in which imaging can be performed and an invalid region in which imaging cannot be performed. A partial region (peripheral portion) of the valid pixel region is masked, so that an image signal cannot be substantially acquired from the partial region. In a case where imaging is performed by using such an image sensor (in a case of global exposure), various phenomena (features) appear in a captured image. Note that, in the present embodiment, a period during which an image is not displayed on a screen is defined as a global exposure period, but an idea of the present embodiment is not limited to this case.

### <Basic concept of dimming control processing according to present embodiment>

In the present embodiment, the light source control unit 1016 (see Fig. 3) alone executes dimming control processing that does not generate both the scanning line noise and the double exposure-like image or makes them less noticeable. Specifically, one frame period is divided into sections of N = 2^{m} (powers of 2 (2, 4, 8, 16, 32, 64, 128,...)), and division that can be implemented by bit shift calculation is only performed as necessary division, so that the dimming control processing can be executed only by the light source control unit (FPGA) 1016 without adding a CPU to save resources. Here, the number of divisions N = 2^{m} of one frame period is determined such that the width (temporal width) of one section after the division falls within the global exposure period. There is no upper limit of the number of divisions. This is because the dimming control processing can be implemented by the bit shift calculation even if the number of divisions is very large, and thus, an excessive load is not applied to the light source control unit 1016.

### <Outline of dimming control processing 1: Example 1>

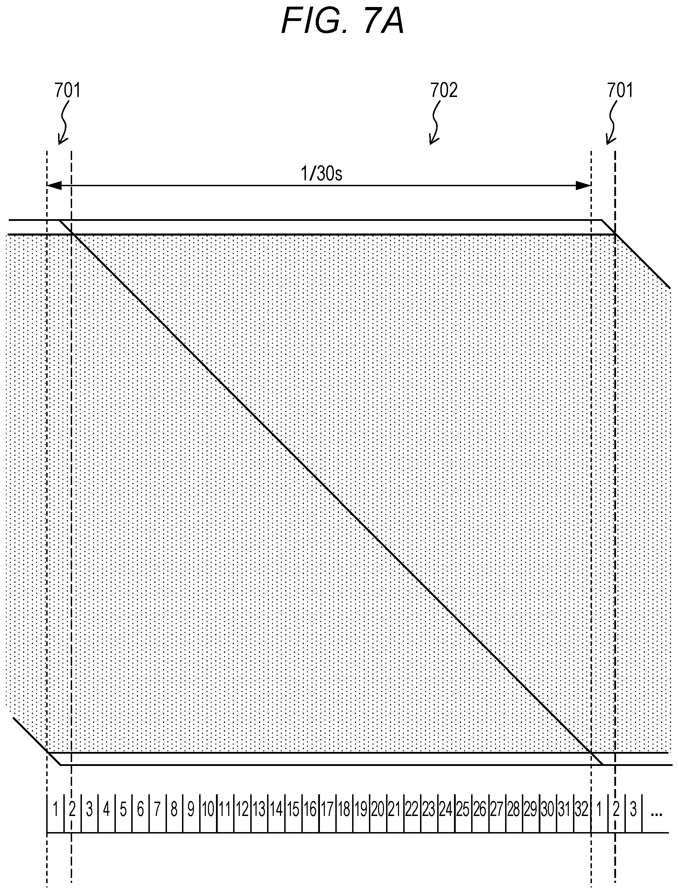

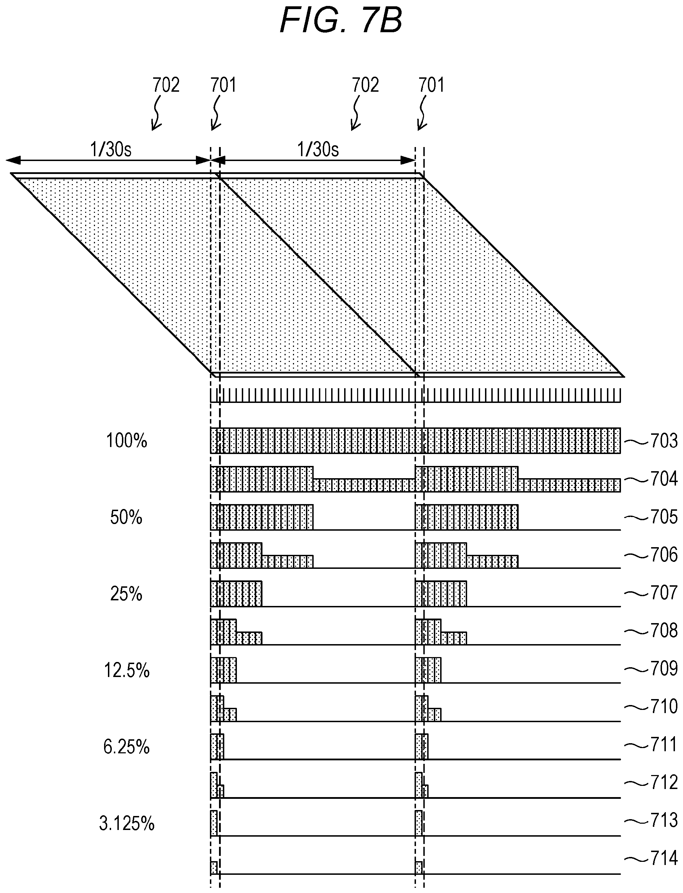

Figs. 7A and 7B are diagrams for describing an outline of dimming control processing 1 (Example 1) according to the present embodiment.

In Figs. 7A and 7B, as an example, one frame period (33.33 ms) of the imaging unit 104 (for example, a CMOS sensor) driven at 30 frames/second (FPS) is divided into 32 (2⁵) sections, and the dimming control processing is executed. In Figs. 7A and 7B, the global exposure period 701 corresponds to a blanking period in which all pixels accumulate effective electric charges in the next reading operation, and is set to, for example, 1.2 ms. After a lapse of 1.2 ms from the start of one frame period, the rolling shutter reading operation of the valid pixels is performed for the remaining 32.13 ms (rolling shutter period 702). Depending on the dimming control processing in the rolling shutter period 702, scanning line noise and a double exposure-like image are undesirably generated. Note that, in Figs. 7A and 7B, when it is necessary to set the light amount to 100%, the light emission intensity is set to the maximum intensity determined from heat at the distal end of the scope or the rated current of the light source, and dimming control is performed such that light is emitted in all of 32 sections, that is, continuous light is generated.

In the dimming control processing according to the present embodiment, the light amount control (increase/decrease) is performed on the basis of a comparison result between the luminance integrated value of a current image (captured image) and the target integrated value determined (instructed) by the operator. However, a sudden change in light amount (brightness) becomes a stress for the operator, and thus, even in a case where light amount control is required, the light amount is controlled not to have a sudden change in brightness by gradually increasing or decreasing the light amount (for example, increasing or decreasing the light amount by 5%).

### (i) When the light amount is controlled within a range of 50% or more and 100% or less

In a case where the light amount (target light amount) is set to A% by the dimming control processing according to Example 1 (50 ≤ A ≤ 100), light emission is controlled with the maximum intensity in the first half sections (for example, 16 sections) of all the sections (for example, 32 sections (N = 32)) of one frame, and light emission is controlled with an intensity of (A × 2 - 100)% in the second half sections (16 sections). That is, dimming control is performed with an emission profile in which light is emitted with the maximum intensity in the first half sections until A = 50%, and in the second half sections, the intensity is gradually decreased and is set to zero (non-emission) when A = 50% (see emission profiles 703 to 705 in Fig. 7B).

### (ii) When the light amount is controlled within a range of 25% or more and less than 50%

In a case where the light amount (target light amount) is set to B% by the dimming control processing according to Example 1 (25 ≤ B < 50), light emission is controlled with the maximum intensity in the first 1/4 sections (8 sections) of all the sections (for example, 32 sections (N = 32)) of one frame, and light emission is controlled with an intensity of (B × 4 - 100)% in the next 1/4 sections (8 sections). In addition, the second half sections (16 sections) of all the sections (32 sections) are maintained at the intensity of zero (turn-off state). That is, dimming control is performed with an emission profile in which light is emitted with the maximum intensity in the first 1/4 sections until B = 25%, and in the next 1/4 sections, the intensity is gradually decreased and is set to zero (non-emission) when B = 25% (see emission profiles 705 to 707 in Fig. 7B).

### (iii) When the light amount is controlled within a range of 100/2ⁿ⁺²% or more and less than 100/2ⁿ⁺¹%

In a case where the light amount (target light amount) is set to C% by the dimming control processing according to Example 1 (100/2ⁿ⁺² ≤ C < 100/2ⁿ⁺¹), light emission is controlled with the maximum intensity in the first N/2ⁿ⁺² sections (for example, if N = 32, 4 sections (when n = 1), 2 sections (when n = 2), or 1 section (when n = 3) (note that n is up to 3 when the number of divisions is 32)) of all the sections (for example, 32 sections (N = 32)) of one frame, and light emission is controlled with an intensity of (C × 2ⁿ⁺² - 100)% in the next N/2ⁿ⁺² sections (if N = 32, 4 sections (when n = 1), 2 sections (when n = 2), or 1 section (when n = 3)). In addition, the remaining (1 - N/2ⁿ⁺²) sections (if N = 32, 24 sections (when n = 1), 28 sections (when n = 2), or 30 sections (when n = 3)) of all of the sections (32 sections) are maintained at the intensity of zero (turn-off state).

For example, dimming control is performed with an emission profile in which light is emitted with the maximum intensity in the first 1/8 sections (4 sections) until C = 12.5%, and in the next 1/8 sections (4 sections), the intensity is gradually decreased and is set to zero (non-emission) when C = 12.5% (see emission profiles 707 to 709 in Fig. 7B). The remaining 3/4 sections (24 sections) are maintained at the intensity of zero (turn-off state).

In addition, dimming control is performed with an emission profile in which light is emitted with the maximum intensity in the first 1/16 sections (2 sections) until C = 6.25%, and in the next 1/16 sections (2 sections), the intensity is gradually decreased and is set to zero (non-emission) when C = 6.25% (see emission profiles 709 to 711 in Fig. 7B). The remaining 7/8 sections (28 sections) are maintained at the intensity of zero (turn-off state).

Further, dimming control is performed with an emission profile in which light is emitted with the maximum intensity in the first 1/32 section (1 section) until C = 3.125%, and in the next 1/32 section (1 section), the intensity is gradually decreased and is set to zero (non-emission) when C = 3.125% (see emission profiles 711 to 713 in Fig. 7B). The remaining 15/16 sections (30 sections) are maintained at the intensity of zero (turn-off state).

### <Details of dimming control processing 1: Example 1>

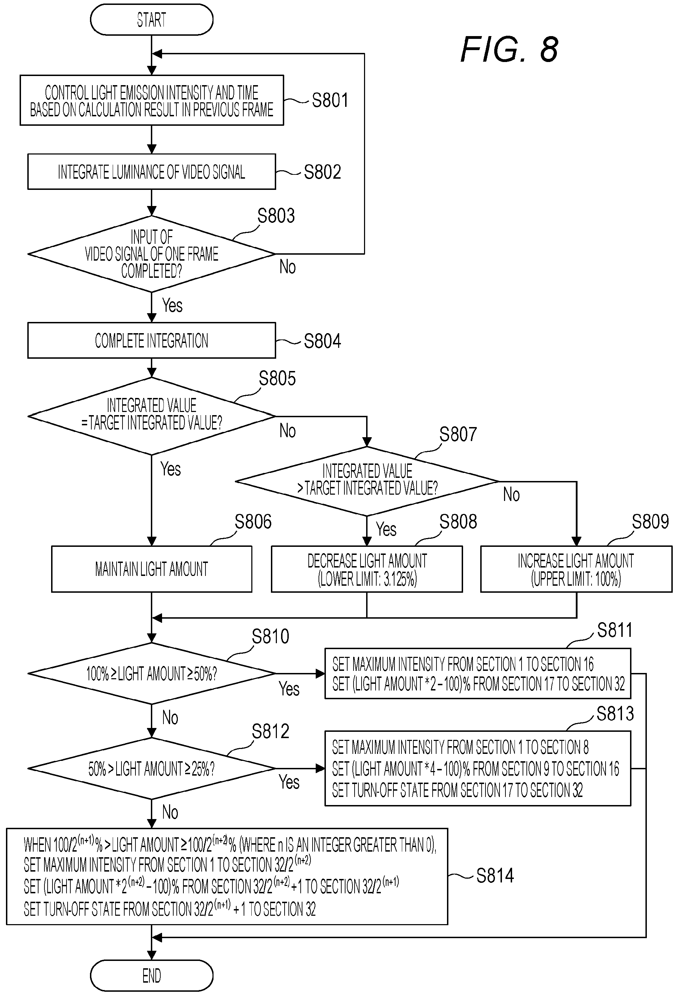

Fig. 8 is a flowchart for describing details of processing for generating an emission profile based on the dimming control processing 1 (Example 1) according to the present embodiment. Here, the part mainly executing the steps of the flowchart may be a CPU or system controller (control device) that is additionally provided or that is included in the processor 200, but when the light source device 101 is provided inside the endoscope device 100 as described above, the part mainly executing the steps is the light source control unit (FPGA) 1016. In the following description, the light source control unit 1016 mainly executes the steps. However, in a case where, for example, the light source device 101 is provided on the processor 200 side, another control device (such as CPU) may mainly execute the steps (the light source control unit 1016 may still mainly execute the steps). Note that the flowchart indicates emission-profile generating processing executed in one frame (current frame), and the emission-profile generating processing is repeated until the target brightness (target integrated value to be described later) designated by the operator is reached. That is, the emission profile generated based on the integrated luminance value of the current frame (generated according to the flowchart of Fig. 8) is used in the dimming control processing for the next frame.

### (i) S801

The light source control unit 1016 irradiates the object with the light emission intensity and the light emission time (the emission profile generated in the previous frame) based on the calculation result in the previous frame, acquires a video signal (a signal for one pixel in the current frame) captured by the imaging unit 104, and converts the video signal into luminance data.

### (ii) S802

The light source control unit 1016 integrates the luminance data (luminance value for one pixel) of the video signal acquired in S801.

### (iii) S803

The light source control unit 1016 determines whether or not the luminance data of the video signal of the current one frame is acquired. When the luminance data for the current one frame is acquired (Yes in S803), the processing proceeds to S804. When all pieces of luminance data for the current one frame have not been acquired (No in S803), the processing returns to S801, and the processes from S801 to S803 are continued until the luminance data of all pixels of the current frame is acquired.

### (iv) S804

The light source control unit 1016 completes the integration of the luminance data. As a result, the light source control unit 1016 acquires the luminance integrated value (for one frame) of the current frame.

### (v) S805

The light source control unit 1016 determines whether or not the luminance integrated value of the current frame acquired in S804 is equal to the target integrated value. Here, the target integrated value is determined according to the brightness level designated (instructed) by the operator through a user interface (UI) of the endoscope device 100 (for example, a table of the target integrated value corresponding to the brightness level is provided in advance).

When the luminance integrated value acquired in S804 is equal to the target integrated value (Yes in S805), the processing proceeds to S806. When the luminance integrated value acquired in S804 is different from the target integrated value (No in S805), the processing proceeds to S807.

### (vi) S806

The light source control unit 1016 maintains the current light amount (the light emission intensity and the light emission time according to the calculation result of the previous frame: emission profile).

### (vii) S807

The light source control unit 1016 determines whether or not the luminance integrated value of the current frame acquired in S804 is greater than the target integrated value. When the luminance integrated value acquired in S804 is greater than the target integrated value (Yes in S807), the processing proceeds to S808. When the luminance integrated value acquired in S804 is not more than the target integrated value (No in S807), the processing proceeds to S809.

### (viii) S808

The light source control unit 1016 decreases the light amount set in the previous frame by a predetermined proportion (light amount corresponding to a fixed value may be subtracted), and determines the light amount (target light amount) in the next frame. The lower limit of the decrease is 3.125%.

### (ix) S809

The light source control unit 1016 increases the light amount set in the previous frame by a predetermined proportion (light amount corresponding to a fixed value may be added), and determines the light amount (target light amount) in the next frame. The upper limit of the increase is 100%.

### (x) S810

The light source control unit 1016 determines whether or not the maintained light amount, the decreased light amount, or the increased light amount (target light amount A) falls within a range of 50% or more and 100% or less (which may be "more than 50%"). When the target light amount A% is within the range of 50% or more and 100% or less (Yes in S810: the light amount at this time is defined as A%), the processing proceeds to S811. When the target light amount A% is out of the range of 50% or more and 100% or less (No in S810), the processing proceeds to S812.

### (xi) S811

The light source control unit 1016 divides one frame time into N, and generates an emission profile for emitting light with the maximum intensity (100%) in the first (first half) N/2 sections (section 1 to section N/2 (if N = 32, from section 1 to section 16)) of the N sections and emitting light with an intensity of (target light amount A × 2 - 100)% in the remaining N/2 sections (section 17 to section 32).

### (xii) S812

The light source control unit 1016 determines whether or not the maintained light amount, the decreased light amount, or the increased light amount (target light amount B) falls within a range of 25% or more and less than 50% (which may be "more than 25%" or "50% or less"). When the target light amount B is within the range of 25% or more and less than 50% (Yes in S812: the light amount at this time is defined as B%), the processing proceeds to S813. When the target light amount B is out of the range of 25% or more and less than 50% (No in S812 (less than 25%)), the processing proceeds to S814.

### (xiii) S813

The light source control unit 1016 generates an emission profile for emitting light with the maximum intensity (100%) in the first N/4 sections (section 1 to section N/4 (if N = 32, from section 1 to section 8)) of the N sections, emitting light with an intensity of (target light amount B × 4 - 100)% in the next N/4 sections (from section (N/4) + 1 to section N/2 (if N = 32, from section 9 to section 16)), and not emitting light (setting a turn-off state) in the remaining N/2 sections (section (N/2) + 1 to section N (if N = 32, from section 17 to section 32)).

### (xiv) S814

In a case where the target light amount is less than 25%, that is, in a case where the target light amount is 100/2ⁿ⁺² or more and less than 100/2ⁿ⁺¹ (the target light amount at this time is defined as C%), the light source control unit 1016 generates an emission profile for emitting light with the maximum intensity (100%) in the first N/2ⁿ⁺² sections (section 1 to section N/2ⁿ⁺²) of all sections (for example, 32 sections (N = 32)) of one frame, emitting light with an intensity of (target light amount C × 2ⁿ⁺² - 100)% in the next N/2ⁿ⁺² sections (section N/2ⁿ⁺² + 1 to section N/2ⁿ⁺¹), and setting the intensity to zero (setting a turn-off state) in the remaining sections (section N/2ⁿ⁺¹ + 1 to section N).

When N = 32, n is from 1 to 3. When n = 1, the target light amount is 12.5% or more and less than 25%, when n = 2, it is 6.25% or more and less than 12.5%, and when n = 3, it is 3.125% or more and less than 6.25%.

(xv) As described above, the dimming control processing (Example 1) for the next frame is executed on the basis of the emission profile generated with the emission-profile generating processing including S801 to S814 for the current frame. When the luminance integrated value obtained by the dimming control processing for the next frame is different from the target integrated value, the emission-profile generating processing in Fig. 8 is further executed. The emission-profile generating processing is repeated until the luminance integrated value in the current frame becomes equal to the target integrated value.

Further, according to Example 1, when the target light amount is between 25% and 100% (when the target light amount is A% or B%), the period in which light is emitted with the maximum intensity corresponds to half of the light emission period, and the period in which light is not emitted with the maximum intensity is always half or less of the entire light emission period (also half or less of the entire division period). For example, under the condition that the target light amount A is 50% or more and 100%, light is emitted with the maximum intensity in a half period of the entire period, and light is emitted with weaker intensity in the remaining period. The large difference in area between the maximum intensity period (for example, 16 periods when N = 32) and the other periods causes the generation of a double exposure-like image. Therefore, by setting the area ratio to less than 1.0, the influence of the double exposure-like image can be decreased.

### <Outline of dimming control processing 2: Example 2>

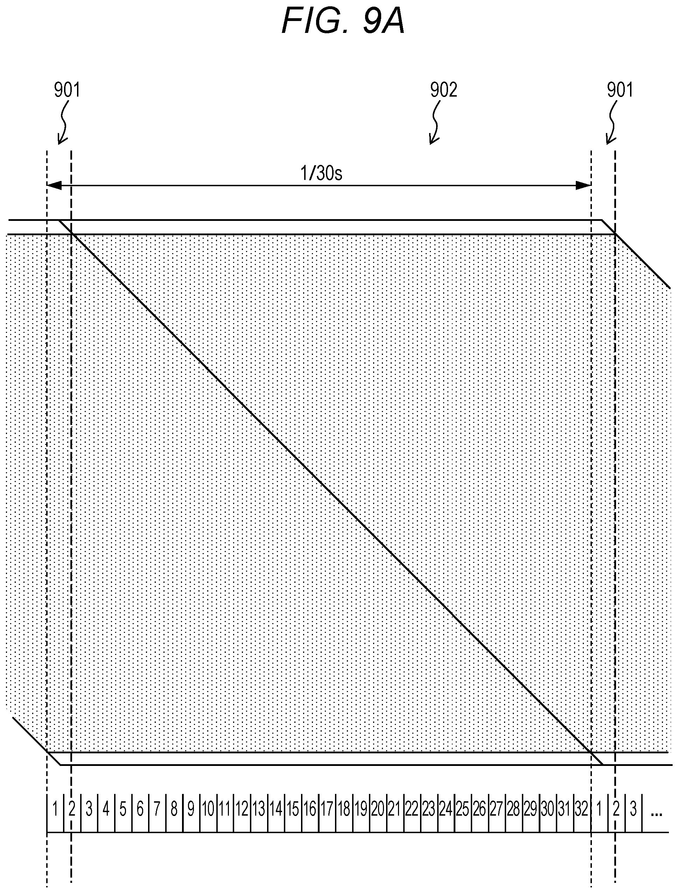

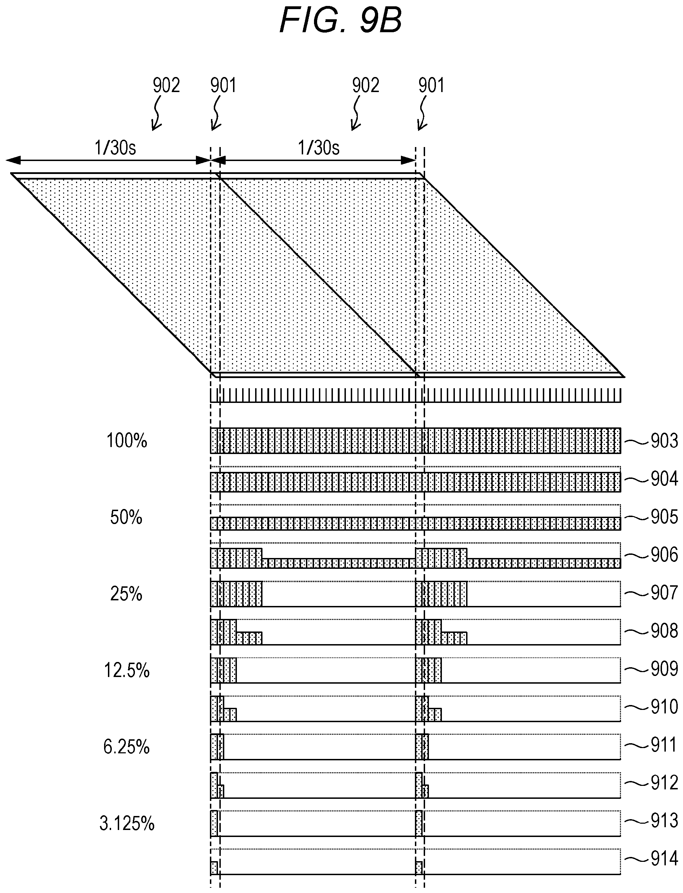

Figs. 9A and 9B are diagrams for describing an outline of dimming control processing 2 (Example 2) according to the present embodiment.

In Figs. 9A and 9B, as an example, one frame period (33.33 ms) of the imaging unit 104 (for example, a CMOS sensor) driven at 30 frames/second (FPS) is divided into 32 (2⁵) sections, and the dimming control processing is executed, as in Figs. 7A and 7B. In Figs. 9A and 9B, the global exposure period 901 also corresponds to a blanking period in which all pixels accumulate effective electric charges in the next reading operation, and is set to, for example, 1.2 ms. After a lapse of 1.2 ms from the start of one frame period, a rolling shutter reading operation of valid pixels is performed for the remaining 32.13 ms (rolling shutter period 902). Depending on the dimming control processing in the rolling shutter period 902, scanning line noise and a double exposure-like image are undesirably generated.

As described above, in the dimming control processing according to the present embodiment, the light amount control (increase/decrease) is performed on the basis of a comparison result between a luminance integrated value of the current image (captured image) and a target integrated value determined (instructed) by the operator. However, a sudden change in light amount (brightness) becomes a stress for the operator, and thus, even in a case where light amount control is required, the light amount is controlled not to have a sudden change in brightness by gradually increasing or decreasing the light amount (for example, increasing or decreasing the light amount by 5%).

### (i) When the light amount is controlled within a range of 50% or more and 100% or less

In a case where the light amount (target light amount) is set to A% by the dimming control processing according to Example 2 (50 ≤ A ≤ 100), light emission is controlled with the intensity of A% in all sections (for example, 32 sections (N = 32)) of one frame, and there is no section where light is not emitted (where turn-off state is set). That is, dimming control is performed with an emission profile in which the intensity is gradually decreased in all sections from section 1 to section N (N = 32) until A = 50%, and when A = 50%, the intensity is halved in all sections (see emission profiles 903 to 705 in Fig. 9B).

### (ii) When the light amount is controlled within a range of 25% or more and less than 50%

In a case where the light amount (target light amount) is set to B% by the dimming control processing according to Example 2 (25 ≤ B < 50), light emission is controlled with an intensity of (150 - B x 2)% in the first 1/4 sections (8 sections) of all the sections (for example, 32 sections (N = 32)) of one frame, and light emission is controlled with an intensity of (B - 25) x 2% in the remaining 3/4 sections (24 sections). That is, dimming control is performed with an emission profile in which, in the first 1/4 sections, the intensity is gradually increased from 50% until B = 25%, and is set to the maximum intensity (100%) when B = 25%, and in the remaining 3/4 sections (24 sections), the intensity is gradually decreased from 50% and is set to zero (non-emission) when B = 25% (see emission profiles 905 to 907 in Fig. 9B).

### (iii) When the light amount is controlled within a range of 100/2ⁿ⁺²% or more and less than 100/2ⁿ⁺¹% (the dimming control processing when the light amount is controlled within a range of less than 25% is similar to that in Example 1)

In a case where the light amount (target light amount) is set to C% by the dimming control processing according to Example 2 (100/2ⁿ⁺² ≤ C < 100/2ⁿ⁺¹), light emission is controlled with the maximum intensity in the first N/2ⁿ⁺² sections (for example, if N = 32, 4 sections (when n = 1), 2 sections (when n = 2), or 1 section (when n = 3) (note that n is up to 3 when the number of divisions is 32)) of all the sections (for example, 32 sections (N = 32)) of one frame, and light emission is controlled with an intensity of (C × 2ⁿ⁺² - 100)% in the next N/2ⁿ⁺² sections (if N = 32, 4 sections (when n = 1), 2 sections (when n = 2), or 1 section (when n = 3)). In addition, the remaining (1 - N/2ⁿ⁺²) sections (if N = 32, 24 sections (when n = 1), 28 sections (when n = 2), or 30 sections (when n = 3)) of all of the sections (32 sections) are maintained at the intensity of zero (turn-off state).

For example, dimming control is performed with an emission profile in which light is emitted with the maximum intensity in the first 1/8 sections (4 sections) until C = 12.5%, and in the next 1/8 sections (4 sections), the intensity is gradually decreased and is set to zero (non-emission) when C = 12.5% (see emission profiles 907 to 909 in Fig. 9B). The remaining 3/4 sections (24 sections) are maintained at the intensity of zero (turn-off state).

In addition, dimming control is performed with an emission profile in which light is emitted with the maximum intensity in the first 1/16 sections (2 sections) until C = 6.25%, and in the next 1/16 sections (2 sections), the intensity is gradually decreased and is set to zero (non-emission) when C = 6.25% (see emission profiles 909 to 911 in Fig. 9B). The remaining 7/8 sections (28 sections) are maintained at the intensity of zero (turn-off state).

Further, dimming control is performed with an emission profile in which light is emitted with the maximum intensity in the first 1/32 section (1 section) until C = 3.125%, and in the next 1/32 section (1 section), the intensity is gradually decreased and is set to zero (non-emission) when C = 3.125% (see emission profiles 911 to 913 in Fig. 9B). The remaining 15/16 sections (30 sections) are maintained at the intensity of zero (turn-off state).

### <Details of dimming control processing 2: Example 2>

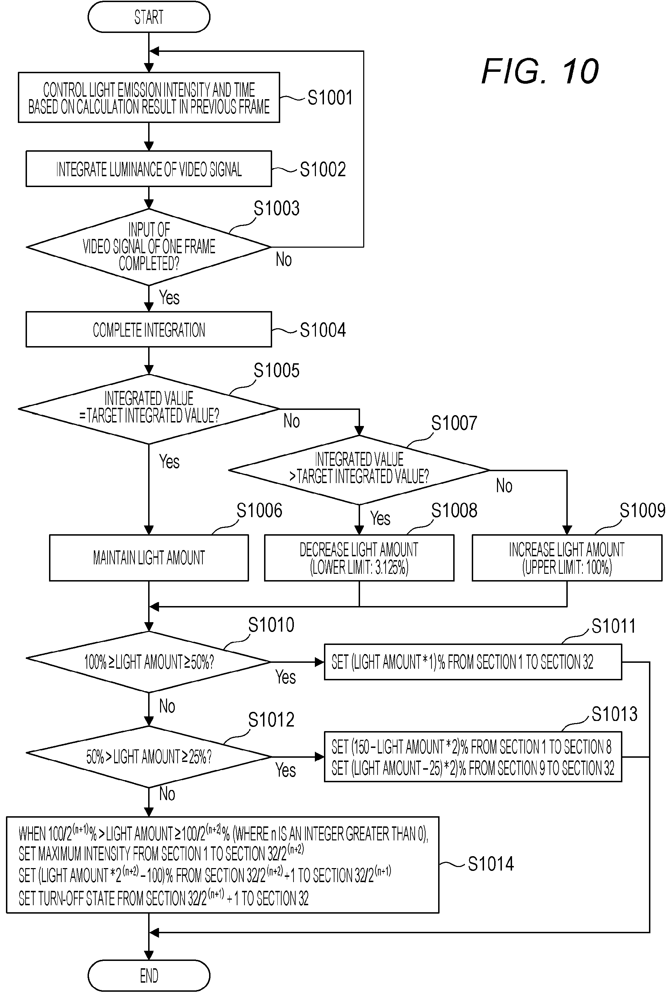

Fig. 10 is a flowchart for describing details of processing for generating an emission profile based on the dimming control processing 1 (Example 2) according to the present embodiment. Here, the part mainly executing the steps of the flowchart may be a CPU or system controller (control device) that is additionally provided or that is included in the processor 200, but when the light source device 101 is provided inside the endoscope device 100 as described above, the part mainly executing the steps is the light source control unit (FPGA) 1016. In the following description, the light source control unit 1016 mainly executes the steps. However, in a case where, for example, the light source device 101 is provided on the processor 200 side, another control device (such as CPU) may mainly execute the steps (the light source control unit 1016 may still mainly execute the steps). Note that the flowchart indicates emission-profile generating processing executed in one frame (current frame), and the emission-profile generating processing is repeated until the target brightness (target integrated value to be described later) designated by the operator is reached. That is, the emission profile generated based on the integrated luminance value of the current frame (generated according to the flowchart of Fig. 10) is used in the dimming control processing for the next frame.

### (i) S1001

The light source control unit 1016 irradiates the object with the light emission intensity and the light emission time (the emission profile generated in the previous frame) based on the calculation result in the previous frame, acquires a video signal (a signal for one pixel in the current frame) captured by the imaging unit 104, and converts the video signal into luminance data.

### (ii) S1002

The light source control unit 1016 integrates the luminance data (luminance value for one pixel) of the video signal acquired in S1001.

### (iii) S1003

The light source control unit 1016 determines whether or not the luminance data of the video signal of the current one frame is acquired. When the luminance data for the current one frame is acquired (Yes in S1003), the processing proceeds to S1004. When all pieces of luminance data for the current one frame have not been acquired (No in S1003), the processing returns to S1001, and the processes from S1001 to S1003 are continued until the luminance data of all pixels of the current frame is acquired.

### (iv) S1004

The light source control unit 1016 completes the integration of the luminance data. As a result, the light source control unit 1016 acquires the luminance integrated value (for one frame) of the current frame.

### (v) S1005

The light source control unit 1016 determines whether or not the luminance integrated value of the current frame acquired in S1004 is equal to the target integrated value. Here, the target integrated value is determined according to the brightness level designated (instructed) by the operator through a user interface (UI) of the endoscope device 100 (for example, a table of the target integrated value corresponding to the brightness level is provided in advance).

When the luminance integrated value acquired in S1004 is equal to the target integrated value (Yes in S1005), the processing proceeds to S1006. When the luminance integrated value acquired in S1004 is different from the target integrated value (No in S1005), the processing proceeds to S1007.

### (vi) S1006

The light source control unit 1016 maintains the current light amount (the light emission intensity and the light emission time according to the calculation result of the previous frame: emission profile).

### (vii) S1007

The light source control unit 1016 determines whether or not the luminance integrated value of the current frame acquired in S1004 is greater than the target integrated value. When the luminance integrated value acquired in S1004 is greater than the target integrated value (Yes in S1007), the processing proceeds to S1008. When the luminance integrated value acquired in S1004 is not more than the target integrated value (No in S1007), the processing proceeds to S1009.

### (viii) S1008

The light source control unit 1016 decreases the light amount set in the previous frame by a predetermined proportion (light amount corresponding to a fixed value may be subtracted), and determines the light amount (target light amount) in the next frame. The lower limit of the decrease is 3.125%.

### (ix) S1009

The light source control unit 1016 increases the light amount set in the previous frame by a predetermined proportion (light amount corresponding to a fixed value may be added), and determines the light amount (target light amount) in the next frame. The upper limit of the increase is 100%.

### (x) S1010

The light source control unit 1016 determines whether or not the maintained light amount, the decreased light amount, or the increased light amount (target light amount) falls within a range of 50% or more and 100% or less (which may be "more than 50%"). When the target light amount is within the range of 50% or more and 100% or less (Yes in S1010: the light amount at this time is defined as A%), the processing proceeds to S1011. When the target light amount is out of the range of 50% or more and 100% or less (No in S1010), the processing proceeds to S1012.

### (xi) S1011

The light source control unit 1016 divides one frame time into N, and generates an emission profile for emitting light with an intensity of the target light amount A% in all of the N sections (if N = 32, from section 1 to section 32).

### (xii) S1012

The light source control unit 1016 determines whether or not the maintained light amount, the decreased light amount, or the increased light amount (target light amount) falls within a range of 25% or more and less than 50% (which may be "more than 25%" or "50% or less"). When the target light amount is within the range of 25% or more and less than 50% (Yes in S1012: the light amount at this time is defined as B%), the processing proceeds to S1013. When the target light amount is outside the range of 25% or more and less than 50% (No in S1012 (less than 25%)), the processing proceeds to S1014.

### (xiii) S1013

The light source control unit 1016 generates an emission profile for emitting light with an intensity of (150 - target light amount B x 2)% in the first N/4 sections (section 1 to section N/4 (if N = 32, from section 1 to section 8)) of the N sections, and emitting light with an intensity of (target light amount B - 25) x 2% in the remaining 3 x N/4 sections (from section (N/4) + 1 to section N (if N = 32, from section 9 to section 32)).

### (xiv) S1014

In a case where the target light amount is less than 25%, that is, in a case where the target light amount is 100/2ⁿ⁺² or more and less than 100/2ⁿ⁺¹ (the target light amount at this time is defined as C%), the light source control unit 1016 generates an emission profile for emitting light with the maximum intensity (100%) in the first N/2ⁿ⁺² sections (section 1 to section N/2ⁿ⁺²) of all sections (for example, 32 sections (N = 32)) of one frame, emitting light with an intensity of (target light amount C × 2ⁿ⁺² - 100)% in the next N/2ⁿ⁺² sections (section N/2ⁿ⁺² + 1 to section N/2ⁿ⁺¹), and setting the intensity to zero (setting a turn-off state) in the remaining sections (section N/2ⁿ⁺¹ + 1 to section N).

When N = 32, n is from 1 to 3. When n = 1, the target light amount is 12.5% or more and less than 25%, when n = 2, it is 6.25% or more and less than 12.5%, and when n = 3, it is 3.125% or more and less than 6.25%.

(xv) As described above, the dimming control processing (Example 2) for the next frame is executed on the basis of the emission profile generated by the emission-profile generating processing including S1001 to S1014 for the current frame. When the luminance integrated value obtained by the dimming control processing for the next frame is different from the target integrated value, the emission-profile generating processing in Fig. 10 is further executed. The emission-profile generating processing is repeated until the luminance integrated value in the current frame becomes equal to the target integrated value.

Further, according to Example 2, when the target light amount is between 25% and 50% (when the target light amount is B%), the area of the period in which light is emitted with (150 - target light amount B × 2)% is sometimes smaller than the area of the period in which light is emitted with (target light amount B - 25) × 2%. That is, the ratio of area = period × intensity may be 1.0 or more, but in this case, the difference in intensity between them is small. For this reason (because light is continuously emitted with substantially the same intensity), the influence (difficulty in viewing the image) caused by the generation of a double exposure-like image is extremely small.

Furthermore, in Example 2, under the condition where the target light amount A is obtained (in the case of 50% ≤ A ≤ 100%), a profile for continuously emitting light is generated. Therefore, it is possible to reduce the discomfort given to the operator and a patient due to the flicker (flickering) of the emitted light when the endoscope device is outside the body cavity.

### <Effects of present embodiment>

According to the present embodiment, it is possible to ensure a sufficient light amount to capture an image of an object while avoiding distortion and artifacts due to rolling shutter. In addition, a continuous change in pulsed light emission period does not occur in the rolling shutter period, and thus, the vertical movement of the horizontal stripes can be made less noticeable.

Furthermore, one frame is divided into dimming sections of 2^{m} (m is an integer of 2 or more), and division that can be implemented by bit shift is only performed as calculation necessary for generating an emission profile, whereby resource saving of the light source device can be achieved only by the light source control unit (FPGA) without using an arithmetic element such as a CPU. Therefore, the calculation resources are limited, and the light source device can be installed on the endoscope device side in a closed space.

### <Specified matters of present disclosure>

### (1) Specified matter 1

A light source device that generates illumination light to be applied to an object, the light source device including:
a semiconductor light emitting element that emits light; and
a light source control unit that generates an emission profile of the semiconductor light emitting element and drives the semiconductor light emitting element on the basis of the emission profile, wherein
the light source control unit executes
   processing of dividing one frame period in an image signal acquired by an image sensor into N (N is an integer of 2^{m}, m being an integer of 2 or more) dimming sections,
   processing of generating an emission profile for controlling light emission intensity in units of 2^{k} (k is an integer from 0 to m) dimming sections included in the dimming sections according to a target light amount in the one frame period, and
   processing of performing dimming control for a next frame on the basis of the emission profile.

### (2) Specified matter 2

The light source device according to Specified matter 1, wherein
the light source control unit divides the one frame period such that one of the dimming sections is smaller than a global exposure period.

### (3) Specified matter 3

The light source device according to Specified matter 1, wherein
the light source control unit determines the target light amount by comparing a luminance integrated value of a video signal of a current frame acquired using an emission profile of a previous frame with a target luminance integrated value that has been input.

### (4) Specified matter 4

The light source device according to Specified matter **3,** wherein
the light source control unit performs dimming control so that the light amount gradually reaches the target luminance integrated value by increasing or decreasing the light amount by a predetermined amount or a predetermined proportion when the luminance integrated value of the current frame is different from the target luminance integrated value.

### (5) Specified matter 5

The light source device according to Specified matter 1, wherein,
when the target light amount is within a range of 50% or more and 100% or less, the light source control unit generates the emission profile for emitting light with a maximum intensity from the dimming section 1 to the dimming section N/2 and emitting light with an intensity of (target light amount × 2 - 100)% from the dimming section (N/2) + 1 to the dimming section N.

### (6) Specified matter 6

The light source device according to Specified matter 5, wherein,
when the target light amount is within a range of 25% or more and less than 50%, the light source control unit generates the emission profile for emitting light with the maximum intensity from the dimming section 1 to the dimming section N/4, emitting light with an intensity of (target light amount × 4 - 100)% from the dimming section (N/4) + 1 to the dimming section N/2, and not emitting light in the remaining dimming sections.

### (7) Specified matter 7

The light source device according to Specified matter 6, wherein,
when the target light amount is within a range of 100/2ⁿ⁺²% or more and less than 100/2ⁿ⁺¹% (n is an integer from 1 to m - 2), the light source control unit generates the emission profile for emitting light with the maximum intensity from the dimming section 1 to the dimming section N/2ⁿ⁺², emitting light with an intensity of (target light amount × 2ⁿ⁺² - 100)% from the dimming section (N/2ⁿ⁺²) + 1 to the dimming section N/2ⁿ⁺¹, and not emitting light in the remaining dimming sections.

### (8) Specified matter 8

The light source device according to Specified matter 1, wherein,
when the target light amount is within a range of 50% or more and 100% or less, the light source control unit generates the emission profile for emitting light with an intensity of the target light amount from the dimming section 1 to the dimming section N.

### (9) Specified matter 9

The light source device according to Specified matter 8, wherein,
when the target light amount is within a range of 25% or more and less than 50%, the light source control unit generates the emission profile for emitting light with an intensity of (150 - target light amount x 2)% from the dimming section 1 to the dimming section N/4 and emitting light with an intensity of (target light amount - 25) × 2% from the dimming section (N/4) + 1 to the dimming section N.

### (10) Specified matter 10

The light source device according to Specified matter 9, wherein,
when the target light amount is within a range of 100/2ⁿ⁺²% or more and less than 100/2ⁿ⁺¹% (n is an integer from 1 to m - 2), the light source control unit generates the emission profile for emitting light with the maximum intensity from the dimming section 1 to the dimming section N/2ⁿ⁺², emitting light with an intensity of (target light amount × 2ⁿ⁺² - 100)% from the dimming section (N/2ⁿ⁺²) + 1 to the dimming section N/2ⁿ⁺¹, and not emitting light in the remaining dimming sections.

### (11) Specified matter 11

An endoscope system that inserts an endoscope device into a site to be observed and acquires an image of an object, the endoscope system including:
a light source device;
an image sensor that irradiates the object with illumination light emitted from the light source device and generates an image signal by detecting reflection light from the object; and
a processor that processes the image signal to generate an image of the object and displays the generated image on a monitor, wherein
the light source device includes
   a semiconductor light emitting element that emits light, and
   a light source control unit that generates an emission profile of the semiconductor light emitting element and drives the semiconductor light emitting element on the basis of the emission profile, and
   the light source control unit executes
      processing of dividing one frame period in an image signal acquired by the image sensor into N (N is an integer of 2^{m}, m being an integer of 2 or more) dimming sections,
      processing of generating an emission profile for controlling light emission intensity in units of 2^{k} (k is an integer from 0 to m) dimming sections included in the dimming sections according to a target light amount in the one frame period, and
      processing of performing dimming control for a next frame on the basis of the emission profile.

### (12) Specified matter 12

The endoscope system according to Specified matter 11, wherein
the light source control unit divides the one frame period such that one of the dimming sections is smaller than a global exposure period.

### (13) Specified matter 13

The endoscope system according to Specified matter 11, wherein
the light source control unit determines the target light amount by comparing a luminance integrated value of a video signal of a current frame acquired using an emission profile of a previous frame with a target luminance integrated value that has been input.

### (14) Specified matter 14

The endoscope system according to Specified matter 13, wherein
the light source control unit performs dimming control so that the light amount gradually reaches the target luminance integrated value by increasing or decreasing the light amount by a predetermined amount or a predetermined proportion when the luminance integrated value of the current frame is different from the target luminance integrated value.

### (15) Specified matter 15

The endoscope system according to Specified matter 11, wherein,
when the target light amount is within a range of 50% or more and 100% or less, the light source control unit generates the emission profile for emitting light with a maximum intensity from the dimming section 1 to the dimming section N/2 and emitting light with an intensity of (target light amount × 2 - 100)% from the dimming section (N/2) + 1 to the dimming section N.

### (16) Specified matter 16

The endoscope system according to Specified matter 15, wherein,
when the target light amount is within a range of 25% or more and less than 50%, the light source control unit generates the emission profile for emitting light with the maximum intensity from the dimming section 1 to the dimming section N/4, emitting light with an intensity of (target light amount × 4 - 100)% from the dimming section (N/4) + 1 to the dimming section N/2, and not emitting light in the remaining dimming sections.

### (17) Specified matter 17

The endoscope system according to Specified matter 16, wherein,
when the target light amount is within a range of 100/2ⁿ⁺²% or more and less than 100/2ⁿ⁺¹% (n is an integer from 1 to m - 2), the light source control unit generates the emission profile for emitting light with the maximum intensity from the dimming section 1 to the dimming section N/2ⁿ⁺², emitting light with an intensity of (target light amount × 2ⁿ⁺² - 100)% from the dimming section (N/2ⁿ⁺²) + 1 to the dimming section N/2ⁿ⁺¹, and not emitting light in the remaining dimming sections.

### (18) Specified matter 18

The endoscope system according to Specified matter 11, wherein,
when the target light amount is within a range of 50% or more and 100% or less, the light source control unit generates the emission profile for emitting light with an intensity of the target light amount from the dimming section 1 to the dimming section N.

### (19) Specified matter 19

The endoscope system according to Specified matter 18, wherein,
when the target light amount is within a range of 25% or more and less than 50%, the light source control unit generates the emission profile for emitting light with an intensity of (150 - target light amount x 2)% from the dimming section 1 to the dimming section N/4 and emitting light with an intensity of (target light amount - 25) × 2% from the dimming section (N/4) + 1 to the dimming section N.

### (20) Specified matter 20

The endoscope system according to Specified matter 19, wherein,
when the target light amount is within a range of 100/2ⁿ⁺²% or more and less than 100/2ⁿ⁺¹% (n is an integer from 1 to m - 2), the light source control unit generates the emission profile for emitting light with the maximum intensity from the dimming section 1 to the dimming section N/2ⁿ⁺², emitting light with an intensity of (target light amount × 2ⁿ⁺² - 100)% from the dimming section (N/2ⁿ⁺²) + 1 to the dimming section N/2ⁿ⁺¹, and not emitting light in the remaining dimming sections.

### (21) Specified matter 21

The endoscope system according to Specified matter 11, wherein
the light source device is provided in the endoscope device.

### Reference Signs List

- 1: Endoscope system
- 100: Endoscope device
- 104: Imaging unit
- 200: Processor
- 101: Light source device
- 1011: Green LED
- 1012: Blue LED
- 1013: Red LED
- 1014: Amber LED
- 1015: UV LED
- 1016: Light source control unit
- 1017, 1018: Cross prism
- 201: System controller
- 202: Photometry unit
- 300: Monitor

## Claims

1. A light source device that generates illumination light to be applied to an object, the light source device comprising:
a semiconductor light emitting element that emits light; and
a light source control unit that generates an emission profile of the semiconductor light emitting element and drives the semiconductor light emitting element on the basis of the emission profile, wherein
the light source control unit executes
processing of dividing one frame period in an image signal acquired by an image sensor into N (N is an integer of 2^{m}, m being an integer of 2 or more) dimming sections,
processing of generating an emission profile for controlling light emission intensity in units of 2^{k} (k is an integer from 0 to m) dimming sections included in the dimming sections according to a target light amount in the one frame period, and
processing of performing dimming control for a next frame on the basis of the emission profile.

2. The light source device according to claim 1, wherein
the light source control unit divides the one frame period such that one of the dimming sections is smaller than a global exposure period.

3. The light source device according to claim 1, wherein
the light source control unit determines the target light amount by comparing a luminance integrated value of a video signal of a current frame acquired using an emission profile of a previous frame with a target luminance integrated value that has been input.

4. The light source device according to claim 3, wherein
the light source control unit performs dimming control so that the light amount gradually reaches the target luminance integrated value by increasing or decreasing the light amount by a predetermined amount or a predetermined proportion when the luminance integrated value of the current frame is different from the target luminance integrated value.

5. The light source device according to claim 1, wherein,
when the target light amount is within a range of 50% or more and 100% or less, the light source control unit generates the emission profile for emitting light with a maximum intensity from the dimming section 1 to the dimming section N/2 and emitting light with an intensity of (target light amount × 2 - 100)% from the dimming section (N/2) + 1 to the dimming section N.

6. The light source device according to claim 5, wherein,
when the target light amount is within a range of 25% or more and less than 50%, the light source control unit generates the emission profile for emitting light with the maximum intensity from the dimming section 1 to the dimming section N/4, emitting light with an intensity of (target light amount × 4 - 100)% from the dimming section (N/4) + 1 to the dimming section N/2, and not emitting light in the remaining dimming sections.

7. The light source device according to claim 6, wherein,
when the target light amount is within a range of 100/2ⁿ⁺²% or more and less than 100/2ⁿ⁺¹% (n is an integer from 1 to m - 2), the light source control unit generates the emission profile for emitting light with the maximum intensity from the dimming section 1 to the dimming section N/2ⁿ⁺², emitting light with an intensity of (target light amount × 2ⁿ⁺² - 100)% from the dimming section (N/2ⁿ⁺²) + 1 to the dimming section N/2ⁿ⁺¹, and not emitting light in the remaining dimming sections.

8. The light source device according to claim 1, wherein,
when the target light amount is within a range of 50% or more and 100% or less, the light source control unit generates the emission profile for emitting light with an intensity of the target light amount from the dimming section 1 to the dimming section N.

9. The light source device according to claim 8, wherein,
when the target light amount is within a range of 25% or more and less than 50%, the light source control unit generates the emission profile for emitting light with an intensity of (150 - target light amount x 2)% from the dimming section 1 to the dimming section N/4 and emitting light with an intensity of (target light amount - 25) × 2% from the dimming section (N/4) + 1 to the dimming section N.

10. The light source device according to claim 9, wherein,
when the target light amount is within a range of 100/2ⁿ⁺²% or more and less than 100/2ⁿ⁺¹% (n is an integer from 1 to m - 2), the light source control unit generates the emission profile for emitting light with the maximum intensity from the dimming section 1 to the dimming section N/2ⁿ⁺², emitting light with an intensity of (target light amount × 2ⁿ⁺² - 100)% from the dimming section (N/2ⁿ⁺²) + 1 to the dimming section N/2ⁿ⁺¹, and not emitting light in the remaining dimming sections.

11. An endoscope system that inserts an endoscope device into a site to be observed and acquires an image of an object, the endoscope system comprising:
a light source device;
an image sensor that irradiates the object with illumination light emitted from the light source device and generates an image signal by detecting reflection light from the object; and
a processor that processes the image signal to generate an image of the object and displays the generated image on a monitor, wherein
the light source device includes
a semiconductor light emitting element that emits light, and
a light source control unit that generates an emission profile of the semiconductor light emitting element and drives the semiconductor light emitting element on the basis of the emission profile, and
the light source control unit executes
processing of dividing one frame period in an image signal acquired by the image sensor into N (N is an integer of 2^{m}, m being an integer of 2 or more) dimming sections,
processing of generating an emission profile for controlling light emission intensity in units of 2^{k} (k is an integer from 0 to m) dimming sections included in the dimming sections according to a target light amount in the one frame period, and
processing of performing dimming control for a next frame on the basis of the emission profile.

12. The endoscope system according to claim 11, wherein
the light source control unit divides the one frame period such that one of the dimming sections is smaller than a global exposure period.

13. The endoscope system according to claim 11, wherein
the light source control unit determines the target light amount by comparing a luminance integrated value of a video signal of a current frame acquired using an emission profile of a previous frame with a target luminance integrated value that has been input.

14. The endoscope system according to claim 13, wherein
the light source control unit performs dimming control so that the light amount gradually reaches the target luminance integrated value by increasing or decreasing the light amount by a predetermined amount or a predetermined proportion when the luminance integrated value of the current frame is different from the target luminance integrated value.

15. The endoscope system according to claim 11, wherein,
when the target light amount is within a range of 50% or more and 100% or less, the light source control unit generates the emission profile for emitting light with a maximum intensity from the dimming section 1 to the dimming section N/2 and emitting light with an intensity of (target light amount × 2 - 100)% from the dimming section (N/2) + 1 to the dimming section N.

16. The endoscope system according to claim 15, wherein,
when the target light amount is within a range of 25% or more and less than 50%, the light source control unit generates the emission profile for emitting light with the maximum intensity from the dimming section 1 to the dimming section N/4, emitting light with an intensity of (target light amount × 4 - 100)% from the dimming section (N/4) + 1 to the dimming section N/2, and not emitting light in the remaining dimming sections.

17. The endoscope system according to claim 16, wherein,
when the target light amount is within a range of 100/2ⁿ⁺²% or more and less than 100/2ⁿ⁺¹% (n is an integer from 1 to m - 2), the light source control unit generates the emission profile for emitting light with the maximum intensity from the dimming section 1 to the dimming section N/2ⁿ⁺², emitting light with an intensity of (target light amount × 2ⁿ⁺² - 100)% from the dimming section (N/2ⁿ⁺²) + 1 to the dimming section N/2ⁿ⁺¹, and not emitting light in the remaining dimming sections.

18. The endoscope system according to claim 11, wherein,
when the target light amount is within a range of 50% or more and 100% or less, the light source control unit generates the emission profile for emitting light with an intensity of the target light amount from the dimming section 1 to the dimming section N.

19. The endoscope system according to claim 18, wherein,
when the target light amount is within a range of 25% or more and less than 50%, the light source control unit generates the emission profile for emitting light with an intensity of (150 - target light amount x 2)% from the dimming section 1 to the dimming section N/4 and emitting light with an intensity of (target light amount - 25) × 2% from the dimming section (N/4) + 1 to the dimming section N.

20. The endoscope system according to claim 19, wherein,
when the target light amount is within a range of 100/2ⁿ⁺²% or more and less than 100/2ⁿ⁺¹% (n is an integer from 1 to m - 2), the light source control unit generates the emission profile for emitting light with the maximum intensity from the dimming section 1 to the dimming section N/2ⁿ⁺², emitting light with an intensity of (target light amount × 2ⁿ⁺² - 100)% from the dimming section (N/2ⁿ⁺²) + 1 to the dimming section N/2ⁿ⁺¹, and not emitting light in the remaining dimming sections.

21. The endoscope system according to claim 11, wherein
the light source device is provided in the endoscope device.
